# EUROPÄISCHE PATENTSCHRIFT

(11) **EP 0 480 954 B1**
(45) Veröffentlichungstag und Bekanntmachung des Hinweises auf die Patenterteilung: **14.04.1993**
(21) Anmeldenummer: 90909657.0
(22) Anmeldetag: 12.06.1990
(51) Int. Cl.: A61F 2/44, A61F 2/46

(54) **NUCLEUSPROTHESE MIT EINER EINFÜHRVORRICHTUNG UND SONDE ZUM BESTIMMEN DER BENÖTIGTEN GRÖSSE DER NUCLEUSPROTHESE**
NUCLEUS PROSTHESIS WITH AN INTRODUCTORY DEVICE AND PROBE TO DETERMINE THE SIZE OF NUCLEUS PROSTHESIS REQUIRED
PROTHESE DU NUCLEUS COMPORTANT UN DISPOSITIF D'INTRODUCTION ET UNE SONDE POUR LA DETERMINATION DE LA GRANDEUR REQUISE DE CETTE PROTHESE

(30) Priorität: 06.07.1989 DE 3922203
(43) Veröffentlichungstag der Anmeldung: 22.04.1992
(73) Patentinhaber: NOLDE, Martin, Dr., D-89407 Dillingen (DE); MARKWALDER, Thomas-Marc, Dr. med., CH-3074 Muri/Bern (CH)
(72) Erfinder: NOLDE, Martin, Dr., D-89407 Dillingen (DE); MARKWALDER, Thomas-Marc, Dr. med., CH-3074 Muri/Bern (CH)
(74) Vertreter: Schoppe, Fritz, Dipl.-Ing.
(86) Internationale Anmeldenummer: EP9000925
(87) Internationale Veröffentlichungsnummer: WO9100713

(56) Entgegenhaltungen:
- EP-A- 0 304 305
- WO-A-81/01098
- US-A- 3 875 595
- US-A- 4 863 477

## Beschreibung

Die vorliegende Erfindung betrifft eine Nucleusprothese mit einer Einführvorrichtung zu deren Einführung in einen Zwischenwirbelraum nach dem Oberbegriff des Patentanspruchs 1.

Allgemein befaßt sich die Erfindung mit dem Gebiet der Bandscheibenprothesen bzw. der Prothesen zum Ersatz des nucleus pulposus, mit einer Einführvorrichtung für die Durchführung der Implantation der Prothese zum Ersatz des nucleus pulposus sowie mit einer Sonde zum Bestimmen der geeigneten Größe einer mit Fluid befüllbaren Prothese zum Ersatz des nucleus pulposus.

Nach der operativen Entfernung des natürlichen nucleus pulposus aus dem Zwischenwirbelraum bzw. Bandscheibenraum kommt es häufig zu einer Instabilität des betreffenden Bewegungs-Segmentes und zu Folgebeschwerden aufgrund einer Höhenminderung im vorderen Bereich dieses operiaten Bewegungs-Segmentes. Eine auch heute noch angewendete Operationstechnik bei anhaltenden Beschwerden dieser Art besteht in der Versteifung des betreffenden Segmentes.

Um Abhilfe für die obenbeschriebenen Probleme, die nach Entfernung des natürlichen nucleus pulposus auftreten, zu schaffen, sind bereits verschiedene Nucleusprothesen oder Bandscheibenprothesen vorgeschlagen worden, die in der Patentliteratur näher erläutert sind. Die meisten dieser in der Patentliteratur beschriebenen Nucleusprothesen haben jedoch keinen Eingang in die medizinische Praxis gefunden, so daß die medizinische Fachwelt bis heute dem Einsatz von Bandscheibenprothesen skeptisch gegenübersteht.

Eine gattungsgemäße Nucleusprothese ist bereits aus der US-A-3 875 595 bekannt. Die bekannte Nucleusprothese hat einen mit einem Fluid befüllbaren Hüllkörper, der in einem unbefüllten Zustand dorsal in einer nicht näher beschriebenen Art in den Zwischenwirbelraum einführbar sein soll. Zum Schutz der benachbarten Nerven und Gefäßstrukturen erfolgt die Einführung der erschlafften, unbefüllten Nucleusprothese durch ein transparentes Rohr, das bis an den rückwärtigen Bereich der Wirbelkörper heranreicht und vor dem anulus fibrosus endet. Vor Einführen des Hüllkörpers werden in die Wirbelkörper vom Zwischenwirbelraum aus hohle Zentrierungsstifte eingepreßt, in die Fortsätze des Hüllkörpers zu dessen Positionierung und Lagesicherung eingreifen sollen, wenn dieser mit Fluid befüllt wird. Die Fluidbefüllung dieser bekannten Nucleusprothese erfolgt über eine Röhre, die an einem Ventilbereich des Hüllkörpers mündet. Das Implantationsverfahren für diese bekannte Nucleusprothese ist zwar in den Beschreibungsunterlagen dieser Patentschrift nicht näher erläutert, jedoch läßt eine Analyse der Figuren darauf schließen, daß das Einführen der Nucleusprothese in den Zwischenwirbelraum durch den Operationszugang im anulus fibrosus allein unter Schwerkraftwirkung erfolgen soll, da der erschlaffte, nicht mit Fluid befüllte Hüllkörper keine eigene Formsteifigkeit hat. In diesem Zustand erscheint die nötige hochgenaue Positionierung des Hüllkörpers vor der Fluidbefüllung als praktisch nicht durchführbar, worin von Fachkreisen der Grund dafür gesehen wird, daß diese bekannte Nucleusprothese keinen Eingang in die medizinische Praxis gefunden hat. Ein weiterer Grund dafür, daß sich diese in der Patentliteratur beschriebene Nucleusprothese in der Praxis nicht durchsetzen konnte, mag darin liegen, daß diese Art einer Prothese nur dann ein mechanisch stabiles Verhalten zeigt, wenn der Hüllkörper von dem Fluid prall ausgefüllt wird, was jedoch ohne Deformation des anulus fibrosus nur dann denkbar ist, wenn das Volumen und die Form der Prothese genau mit der Gestalt des Hohlraumes übereinstimmt, der durch Extraktion des prolabierten Nucleus-anulus-fibrosus-Materials und partielle Ausräumung des Zwischenwirbelraumes erzeugt ist. Ist jedoch der Hüllkörper beispielsweise geringfügig größer als der Hohlraum im Zwischenwirbelbereich, so führt dessen Fluidbefüllung nicht zur Erzeugung eines faltenfreien, prall-elastischen Kissens mit einem Verhalten, das dem mechanischen Verhalten des natürlichen nucleus pulposus entspricht.

Aus der EP-A1-0 304 305 ist eine Nucleusprothese bekannt, die zwei fluidbefüllbare Hüllkörper umfaßt, die im Bereich der lateralen Kanten beidseitig der beschädigten Bandscheibe durch zwei Zugänge einsetzbar sind. Ein derartiges zweiteiliges Implantat kann zwangsläufig nicht das gleiche mechanische Verhalten für die federnd schwenkbewegliche Abstützung der beiden Wirbelkörper gegeneinander haben wie der zentral angeordnete, natürliche nucleus pulposus.

Die EP-A1-0 277 282 offenbart eine komplette Bandscheibenprothese, die den gesamten Zwischenwirbelraum ausfüllt und sowohl den nucleus pulposus wie auch den diesen umgebenden anulus fibrosus ersetzt. Diese bekannte Gelenkendoprothese umfaßt feste Lagerschalen, die als Verankerungselemente dienen und aus mehreren Lagen eines metallischen Drahtnetzes bestehen, das beispielsweise aus Reintitan oder einer Titanlegierung bestehen kann. Zwischen diesen Lagerschalen liegt ein kissenartiger, elastischer Hohlkörper mit einem geschlossenen Hohlraum 7, der mit einem fließfähigen, inkompressiblen Medium gefüllt ist. Eine derartige, komplette Bandscheibenprothese, die den gesamten Raum zwischen zwei benachbarten Wirbelkörpern ausfüllt, ist nur ventral mittels eines entsprechend umfangreichen operativen Eingriffes implantierbar.

Im Prüfungsverfahren des der vorliegenden Anmeldung zugrundeliegenden, nunmehr erteilten deutschen Patentes 39 22 203.9 wurde zusätzlich zu den obigen Entgegenhaltungen folgende Druckschrift zum technologischen Hintergrund der Erfindung genannt: DE 37 04 089 A1.

Aus der WO-81/01098 ist eine Sonde zum Bestimmen der benötigten Größe einer zervikalen Dehnung bekannt, bei der ein vorderer Bereich eines Katheders von einem elastischen Ballon umgeben ist, der an einem Mittenbereich des Katheders dichtend befestigt ist. Die Sonde umfaßt eine Zuführvorrichtung für Druckgas zum Aufblasen des Ballons. Für die Bestimmung eines Hohlraumes im Zwischenwirbelbereich und somit für die Bestimmung der benötigten Größe einer Nucleusprothese ist diese Sonde nicht geeignet, da durch Luft, Flüssigkeiten und Blut innerhalb des auszumessenden Hohlraumes eine nicht zutreffende, nämlich zu geringe Größe des Hohlraumes ermittelt würde.

Gegenüber diesem Stand der Technik liegt der vorliegenden Erfindung die Aufgabe zugrunde, eine Nucleusprothese der eingangs genannten Art so weiterzubilden, daß sie vergleichsweise einfach und zuverlässig von dorsal implantierbar ist und dennoch ein mechanisch der natürlichen Bandscheibe entsprechendes Verhalten aufweist.

Diese Aufgabe wird bei einer Nucleusprothese nach dem Oberbegriff des Patentanspruchs 1 durch das im kennzeichnenden Teil des Patentanspruchs 1 angegebene Merkmal gelöst.

Der Erfindung liegt die Erkenntnis zugrunde, daß eine Nucleusprothese der eingangs beschriebenen Art, die einen mit Fluid befüllbaren Hüllkörper aufweist, der in einem ungefüllten oder nur teilweise mit Fluid befüllten Zustand in den Zwischenwirbelraum eingeführt wird und erst dort mit Fluid befüllt wird, dann hinreichend sicher positionierbar und sicher handhabbar wird, wenn die Einführvorrichtung ein die Nucleusprothese in deren ungefüllten oder nur teilweise befüllten Zustand bei ihrer Einführung in den Zwischenwirbelraum umschließendes und in seinen Querschnittsabmessungen an die Abmessungen des Operationszuganges im anulus fibrosus angepaßtes Einführrohr aufweist, mit dem die Nucleusprothese im Zwischenwirbelraum positionierbar ist.

Ferner liegt der Erfindung die Erkenntnis zugrunde, daß bei einer gattungsgemäßen Sonde dann eine zutreffende Ausmessung des Hohlraumes im Zwischenwirbelbereich erfolgen kann, wenn der Ballon einen derartigen Elastizitätsverlauf hat, daß er im Bereich der Kanülenspitze leichter dehnbar als im Bereich der Befestigung an der Kanüle ist. Hierdurch wird sich der Ballon bei seiner Fluidbefüllung an die Wandbereiche des Hohlraumes im Zwischenwirbelbereich anlegen, wodurch Luft, Flüssigkeiten und Blut aus dem auszumessenden Hohlraum während des Befüllens des Ballons verdrängt werden.

Bevorzugte Weiterbildungen sind in den Unteransprüchen angegeben.

Eine bevorzugte Ausführungsform der Sonde zum Bestimmen der benötigten Größe einer mit Fluid befüllbaren Nucleusprothese sowie einer Ausführungsform der Nucleusprothese selbst mit einer Einführvorrichtung zu ihrer Einführung in den Zwischenwirbelraum werde nachfolgend unter Bezugnahme auf die Zeichnungen näher erläutert.

Es zeigen:
- Fig. 1: eine Ausführungsform der erfindungsgemäßen Sonde bei ihrer Einführung in einen Zwischenwirbelraum, teilweise in Schnittdarstellung;
- Fig. 2: eine Längsschnittdarstellung eines Abschnittes der Ausführungsform der Sonde gem. Fig. 1 im Bereich ihrer Manschette;
- Fig. 3: eine Querschnittsdarstellung durch die Ausführungsform der Sonde gem. Fig. 1 im Bereich der Manschette;
- Fig. 4: eine Ausführungsform der Nucleusprothese im unbefüllten Zustand mit einer Ausführungsform einer Einführvorrichtung;
- Fig. 5: eine Darstellung der Einführung der Nucleusprothese und der Einführvorrichtung in den Hohlraum im Zwischenwirbelraum;
- Fig. 6: eine Darstellung der Nucleusprothese nach Zurückziehen der Einführvorrichtung während ihres Befüllens, teilweise in Schnittdarstellung;
- Fig. 7: eine schematische Darstellung einer Ausführungsform einer Befüllungsvorrichtung für die Nucleusprothese;
- Fig. 8: eine Horizontalschnittdarstellung durch die Nucleusprothese gegen Ende des Befüllungsvorganges bei einem noch angesetzten Befüllungsrohr;
- Fig. 9: eine der Fig. 8 entsprechende Darstellung der Nucleusprothese allein bei abgenommenem Füllungsrohr und eingesetzter Imbusschraube; und
- Fig.10: eine weitere Darstellung in der vertikalen Ebene der Nucleusprothese nach Abschluß des Implantierens.

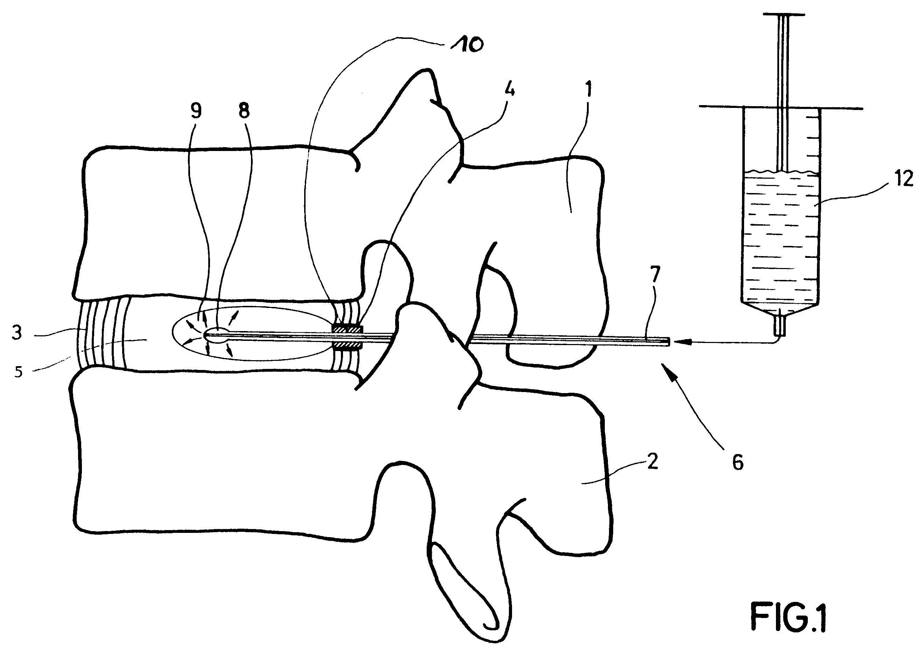

Wie in Fig. 1 gezeigt ist, liegt zwischen zwei Wirbelkörpern 1, 2 ein anulus fibrosus 3, der normalerweise den (in Fig. 1 nicht gezeigten) nucleus pulposus umschließt. Nach erfolgter Extraktion des prolabierten Nucleus-pulposus-Materiales und Anulus-fibrosus-Materiales durch den dorsalen Operationszugang 4 ensteht im Zwischenwirbelbereich ein Hohlraum oder Zwischenbelraum 5. Zum Bestimmen der benötigten Größe einer mit Fluid befüllbaren Nucleusprothese (nicht dargestellt), die in einer Ausführungsform später näher erläutert wird, wird die in Fig. 1 gezeigte Ausführungsform einer erfindungsgemäßen Sonde verwendet, die in ihrer Gesamtheit mit dem Bezugszeichen 6 bezeichnet ist. Die Sonde umfaßt eine Kanüle 7, deren Außendurchmesser geringer ist als die Abmessung des Operationszuganges 4 durch den anulus fibrosus 3, und die an ihrem vorderen Ende eine ballige Abrundung 8 aufweist. Die Sonde 6 ist an ihrem vorderen Bereich von einem elastischen Ballon 9 umgeben, der mit der Kanüle 7 über eine konische oder kegelstumpfförmige Manschette 10 in dichtender Verbindung steht.

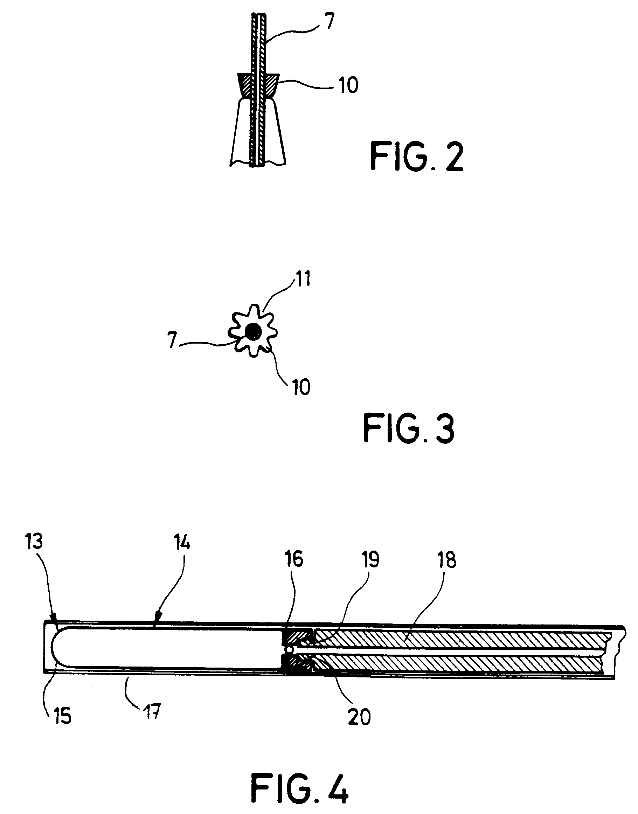

Wie insbesondere in Fig. 2 zu erkennen ist, nimmt der Durchmesser der Manschette 10 in Richtung zu dem hinteren, von der balligen Abrundung 8 abgewandten Ende zu.

Bei der gezeigten Ausführungsform hat die Manschette eine Querschnittsform, die als zackenförmig oder sternförmig ausgestaltet sein kann, wie dies insbesondere der Fig. 3 zu entnehmen ist. Hierdurch wird eine Mehrzahl von Öffnungen 11 festgelegt, die auch bei festem Anliegen der Peripherie der Manschette 10 am Operationszugang 4 ein in Achsrichtung der Kanüle 7 erfolgendes Durchtreten von Luft, Blut oder sonstigen Flüssigkeiten aus dem Zwischenwirbelraum 5 ermöglichen. Der Ballon 9 weist vorzugsweise einen derartigen stetigen Elastizitätsverlauf auf, daß dieser im Kanülenspitzenbereich leichter dehnbar ist als im Bereich seiner Befestigung an der Kanüle 7.

Die Sonde umfaßt ferner eine Fluidzuführvorrichtung 12, die bei der in Fig. 1 gezeigten bevorzugten Ausführungsform durch eine handelsübliche Spritze gebildet wird.

Nach Plazierung der Sonde 6 innerhalb des vom anulus fibrosus 3 umschlossenen Zwischenwirbelraumes 5 wird der Ballon 9 durch Betätigung der Spritze 12 mit einem Fluid gefüllt, das vorzugsweise ein sterilisierbares Röntgenkontrastmittel ist. Die Füllung erfolgt, bis ein vorbestimmter Füllungsgrad und Füllungsdruck erreicht ist. Durch den obenerläuterten Elastizitätsverlauf des Ballones 9 wird im Zwischenwirbelraum befindliche Luft oder Blut oder sonstige Flüssigkeiten zum Operationszugang 4 gedrängt, wo diese Medien durch die Öffnungen 11 heraustreten. Nach Erreichen des vorbestimmten Füllungsdruckes, der bei Verwenden einer Spritze 12 durch den von Hand spürbaren Gegendruck der Spritzenbetätigung bestimmbar ist, wird das injizierte Volumen des Röntgenkontrastmittels abgelesen. Aus diesem Volumen zuzüglich des im Hohlraum befindlichen Volumenanteiles des Ballones 9 sowie der Kanüle 7 abzüglich des in der zuführenden Kanüle 7 befindlichen Flüssigkeitvolumens kann das Hohlraumvolumen im Zwischenwirbelraum 5 und damit die erforderliche Prothesengröße abgeleitet werden. Anschließend wird durch absaugende Betätigung der Spritze 12 die Flüssigkeit aus dem System abgezogen und die Sonde 6 durch den Operationszugang 4 aus dem Zwischenwirbelraum 5 herausgezogen.

Nachfolgend wird unter Bezugnahme auf Fig. 4 eine bevorzugte Ausführungsform der erfindungsgemäßen Nucleusprothese, die in ihrer Gesamtheit mit dem Bezugszeichen 13 bezeichnet ist, zusammen mit einer Einführvorrichtung 14 zu deren Einführung in den Zwischenwirbelraum 5 näher erläutert. Die Nucleusprothese 13 umfaßt einen Hüllkörper 15, der in Fig. 4 in seinem unbefüllten Zustand gezeigt ist und ein Ventil 16 aufweist, über das er mit einem Fluid befüllt werden kann. Wie in Fig. 4 gezeigt ist, ist die Einführvorrichtung als ein die Nucleusprothese 13 in ihrem unbefüllten Zustand umschließendes Einführrohr 17 ausgebildet, das zu deren Einführung in den Zwischenwirbelraum 5 dient und in seinen Querschnittsaußenabmessungen an die Abmessungen des Operationszuganges 4 im anulus fibrosus 3 derart angepaßt ist, daß dessen Durchmesser bei im querschnitt kreisförmiger Ausgestaltung des Einführrohres 17 kleiner ist als die kleinste Abmessung eines üblichen Operationszuganges 4. Die Einführvorrichtung 14 umfaßt ferner eine als Befüllungsrohr 18 ausgestaltete Ausschubvorrichtung, mit der die Nucleusprothese 13 aus dem Einführrohr 17 ausschiebbar ist. Das Befüllungsrohr 18 hat ein Außengewinde 19. Die Nucleusprothese 13 hat im Bereich des Ventiles 16 ein zu diesem Außengewinde 19 passendes Innengewinde 20.

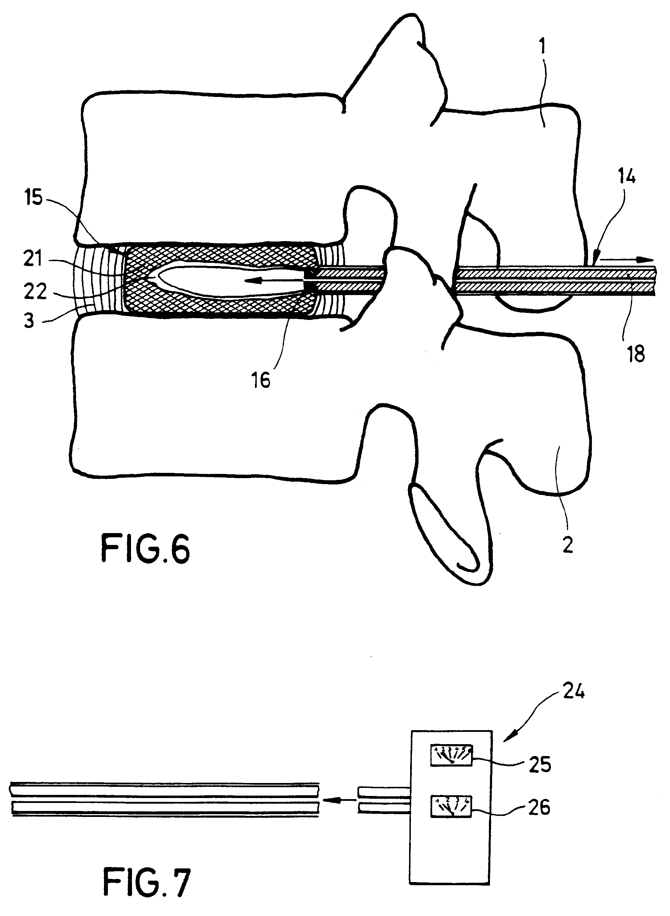

Der Hüllkörper 15 ist zweischichtig ausgeführt. Die äußere Hüllkörperschicht (vgl. Fig. 6) besteht aus einem hochfesten, mit dem menschlichen Körpergewebe verträglichen Draht- oder Fasergewebe, das vorzugsweise aus einem Titangewebe besteht. Die innere Hüllkörperschicht (vgl. Fig. 6) besteht aus einem fluidundurchlässigen Kunststoffgewebe, das bei der bevorzugten Ausgestaltung eine Goretex-Gewebeschicht ist.

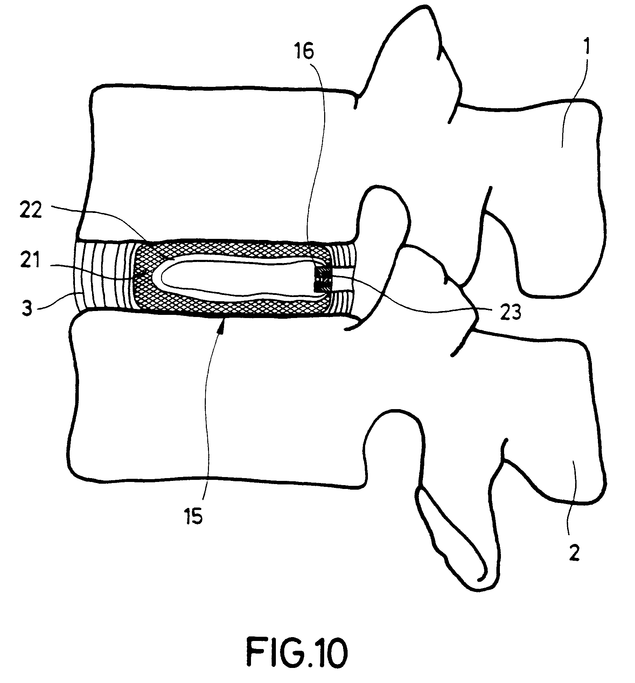

Wie in den Fig. 9 und 10 zu erkennen ist, ist das Ventil 16 derartig innerhalb des Hüllkörpers 15 angeordnet, daß das Ventil 16 stufenfrei in die sphärische Oberfläche des Hüllkörpers 15 in dessen mit Fluid vollständig befüllten Zustand übergeht. Die Nucleusprothese 13 umfaßt ferner eine zu dem Innengewinde 20 passende Imbusschraube 23, mit der das Innengewinde für die Abdeckung desselben verschraubbar ist. Die Imbusschraube ist derart ausgestaltet, daß sie ebenfalls glatt und stufenfrei in die durch den Hüllkörper 15 und das Ventil 16 definierte sphärische Fläche übergeht. Die Imbusschraube bewirkt, daß ein Einwachsen von Narbengewebe oder Eindringen von Verschmutzungen in den Bereich des Ventiles verhindert wird, so daß die erfindungsgemäße Nucleusprothese 13 auch lange Zeit nach ihrer Implantation problemlos geöffnet, geleert oder stärker befüllt werden kann.

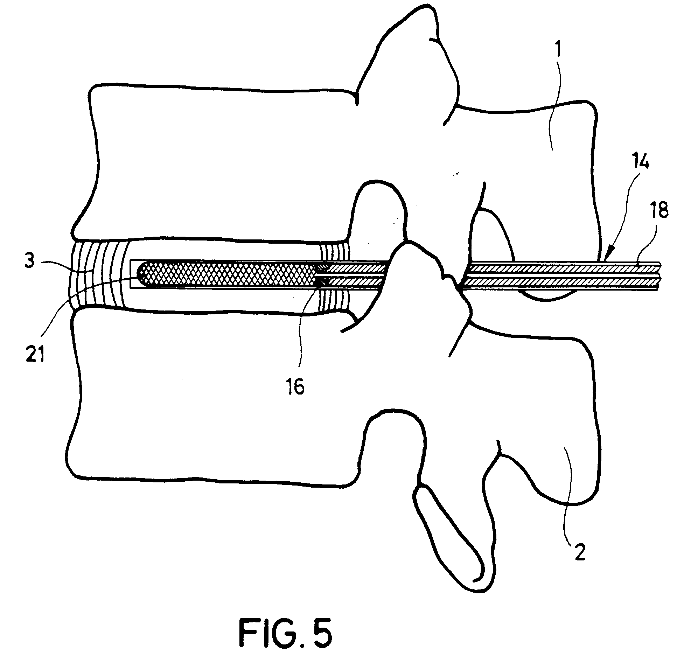

Nachfolgend wird die Implantation der erfindungsgemäßen Nucleusprothese mittels der erfindungsgemäßen Einführvorrichtung unter Bezugnahme auf die Fig. 5 - 10 näher erläutert.

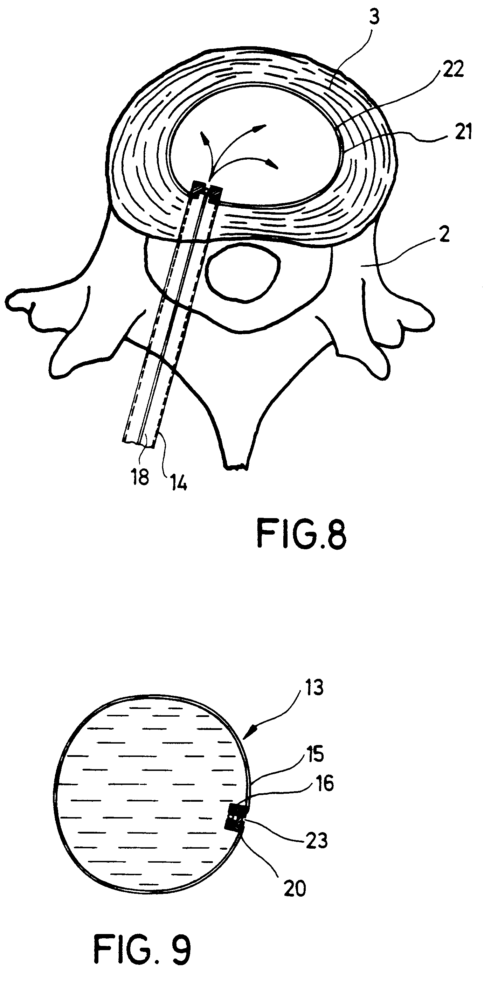

Wie in Fig. 5 gezeigt ist, wird nach den Operationsschritten der Entfernung des prolabierten Nucleuspulposus-Materiales und des Anulus-Fibrosus-Materiales und der partiellen Ausräumung des Zwischenwirbelraumes 5 sowie nach der eingangs beschriebenen Bestimmung der geeigneten Prothesengröße eine Nucleusprothese 13 von derartiger Größe mittels der Einführvorrichtung 14 durch den Operationszugang 4 im Anulus-Fibrosus 3 in den Zwischenwirbelraum 5 eingeführt. Die exakte Plazierung der Prothese 13 wird mittels eines Röntgenmonitores überwacht. Nach optimierter Plazierung der Nucleusprothese 13 wird das Einführrohr 17 zurückgezogen, wobei die Nucleusprothese an einem unerwünschten Mitgleiten durch Festhalten des Befüllungsrohres 18 gehindert wird. In einem nächsten Schritt wird der Hüllkörper 15 durch das Befüllungsrohr 18 unter Öffnung des Ventiles 16 mit einer sterilen, biokompatiblen Flüssigkeit bis zum Erreichen eines vorbestimmten Druckes befüllt. Während dieser Befüllung legt sich der Hüllkörper 15 glatt an die Wandungen des Zwischenwirbelraumes 5 bzw. den Anulus-Fibrosus 3 an, wie in der Vertikalteilschnittdarstellung gemäß Fig. 6 und der Horizontalschnittdarstellung gemäß Fig.8 zu erkennen ist.

Die Befüllung kann mit einer maschinellen Druckpreßvorrichtung vorgenommen werden, die in Fig. 7 in ihrer Gesamtheit mit dem Bezugszeichen 24 bezeichnet ist. Vorzugsweise weist eine derartige, ansich bekannte Druckpreßvorrichtung ein Volumeter 25 und ein Manometer 26 auf. Nach Distension der Nucleusprothese 13 erfolgt eine nochmalige Röntgenkontrolle zur Sicherstellung der gewünschten Plazierung der Nucleusprothese. Anschließend erfolgt das Abschrauben des Befüllungsrohres 18 von der Nucleusprothese, wobei das zwischenzeitlich offenliegende Innengewinde 20 des Ventiles 16 zwangsläufig im Bereich des Operationszuganges 4 gut zugänglich bleibt. Nunmehr wird das Innengewinde 20 mittels der Imbusschraube 23 verschlossen, wie dies in Fig. 10 zu erkennen ist.

Mit dem Verschließen des Ventiles 16 der Nucleusprothese 13 ist die eigentliche Implantation der erfindungsgemäßen Prothese abgeschlossen.

## Patentansprüche

1. Nucleusprothese mit einer Einführvorrichtung (14) zu deren Einführung in einen Zwischenwirbelraum,
wobei die Nucleusprothese (13) einen mit einem Fluid befüllbaren Hüllkörper (15) aufweist, der in einem unbefüllten oder nur teilweise mit Fluid befüllten Zustand durch die Einführvorrichtung in den Zwischenwirbelraum einführbar und dort mit dem Fluid befüllbar ist und ein Ventil (16) aufweist, das in dem Hüllkörper (15) angeordnet ist,
wobei die Einführvorrichtung (14) ein den Hüllkörper (15) in dessen unbefüllten oder nur teilweise befüllten Zustand bei seiner Einführung in den Zwischenwirbelraum (5) umschließendes und in seinen Querschnittsaußenabmessungen an die Abmessungen des Operationszuganges (4) im Anulus-Fibrosus (3) angepaßtes Einführrohr (17) aufweist, mit dem der Hüllkörper (15) im Zwischenwirbelraum (5) positionierbar ist,
dadurch gekennzeichnet,
daß innerhalb des Einführrohres (17) ein Befüllungsrohr (18) vorgesehen ist, das mit dem Hüllkörper (15) im Bereich seines Ventiles (16) zum Ausschieben des Hüllkörpers (15) aus dem Einführrohr (17) in seinen im Zwischenwirbelraum (5) angeordneten Zustand und zum Fluidbefüllen des Hüllkörpers (15) in einen lösbaren Eingriff bringbar ist.

2. Nucleusprothese mit Einführvorrichtung nach Anspruch 1, dadurch gekennzeichnet,
daß das Befüllungsrohr (18) ein Außengewinde (19) aufweist,
daß die Nucleusprothese (13) im Bereich des Ventiles (16) ein zu dem Außengewinde (19) passendes Innengewinde (20) aufweist und
daß die Nucleusprothese (13) eine zu dem Innengewinde (20) passende Schraube (23) hat, mit der dieses zum Abdecken desselben und des Ventiles (16) nach der Fluidbefüllung der Nucleusprothese (13) und nach dem Abschrauben des Befüllungsrohres (18) verschraubbar ist.

3. Nucleusprothese mit Einführvorrichtung nach Anspruch 2, dadurch gekennzeichnet,
daß die Schraube als Imbusschraube (23) ausgebildet ist.

4. Nucleusprothese mit Einführvorrichtung nach einem der Ansprüche 1 - 3, dadurch gekennzeichnet,
daß das Ventil (16) stufenfrei in die sphärische Oberfläche des Hüllkörpers (15) in dessen mit Fluid vollständig befüllten Zustand übergeht.

5. Nucleusprothese mit Einführvorrichtung nach einem der Ansprüche 1 - 4, dadurch gekennzeichnet,
daß der Hüllkörper (15) zweischichtig ausgeführt ist,
daß die äußere Hüllkörperschicht (21) aus einem hochfesten, mit dem menschlichen Körpergewebe verträglichen Draht- oder Fasergewebe besteht, und
daß die innere Hüllkörperschicht (22) aus einem gegenüber dem verwendeten Fluid undurchlässigen Material besteht.

6. Nucleusprothese mit Einführvorrichtung nach Anspruch 5, dadurch gekennzeichnet,
daß die äußere Hüllkörperschicht aus einem Titangewebe (21) besteht.

7. Nucleusprothese mit Einführvorrichtung nach Anspruch 5 oder 6, dadurch gekennzeichnet,
daß die innere Hüllkörperschicht aus einem fluidundurchlässigen Kunststoffgewebe, das bezüglich seiner Eigenschaften dem unter dem Warenzeichen Goretex erhältlichen Gewebe entspricht, besteht.

8. Sonde zum Bestimmen der benötigten Größe einer mit Fluid befüllbaren Nucleusprothese nach einem der Ansprüche 1 bis 7 mit einer Kanüle (7), einem einen vorderen Bereich der Kanüle (7) umgebenden elastischen Ballon (9), der an der Kanüle (7) dichtend befestigt ist und einer Fluidzuführvorrichtung (12) zum Zuführen einer volumenmäßig bestimmbaren Fluidmenge durch die Kanüle (7) zu dem Innenbereich des Ballons (9),
dadurch gekennzeichnet,
daß der Außendurchmesser der Kanüle (7) geringer als die Abmessung eines Operationszuganges (4) im Anulus-Fibrosus (3) ist, und
daß der elastische Ballon (9) im Kanülenspitzenbereich leichter dehnbar als im Bereich seiner Befestigung an der Kanüle (7) ist.

9. Sonde nach Anspruch 8, dadurch gekennzeichnet,
daß die Fluidzuführvorrichtung durch eine mit der Kanüle (7) verbundene Spritze (12) gebildet ist.

10. Sonde nach Anspruch 8 oder 9, dadurch gekennzeichnet,
daß die Kanüle (7) an ihrem ballonseitigen Ende eine ballige Abrundung (8) aufweist.

11. Sonde nach einem der Ansprüche 8 - 10, gekennzeichnet-durch
eine konische oder kegelstumpfförmige Manschette (10), die die Kanüle (7) im Befestigungsbereich des Ballones (9) umgibt.

12. Sonde nach Anspruch 11, dadurch gekennzeichnet,
daß die Manschette (10) wenigstens eine Durchgangsöffnung (11) in Richtung der Kanülenlängserstreckung aufweist.

## Claims

1. A nucleus prothesis provided with an inserting device (14) for inserting it into an intervertebral space,
said nucleus prothesis (13) comprising an enveloping body (15) which can be filled with a fluid, and said enveloping body, when not yet filled or only partially filled with fluid, being adapted to be introduced by means of the inserting device into the intervertebral space where it can then be filled with said fluid, said nucleus prothesis (13) being provided with a valve (16) arranged in the enveloping body (15), said inserting device (14) including an insertion tube (17), which encloses the nucleus prothesis (13) in its unfilled or only partially filled condition when said nucleus prothesis is being inserted into the intervertebral space (5) and the outer cross-sectional dimensions of which are adapted to the dimensions of the operating access (4) in the anulus fibrosus (3), said insertion tube (17) being adapted to be used for positioning the nucleus prothesis (13) in said intervertebral space (5), characterized in
that a filling tube (18) is arranged within the insertion tube (17), the filling tube (18) is adapted to be brought into releasable engagement with the enveloping body (15) in the area of its valve (16) for expelling the enveloping body (15) from the insertion tube (17) in its condition arranged in the intervertebral space and for effecting a supply of fluid to the enveloping body (15).

2. A nucleus prothesis provided with an inserting device according to claim 1, characterized in
that the filling tube (18) is provided with an external thread (19),
that, in the area of the valve (16), the nucleus prothesis (13) is provided with an internal thread (20) matching with said external thread (19), and
that the nucleus prothesis (13) is provided with a screw (23), which matches with said internal thread (20) and to which said internal thread can be screw-fastened for covering the same and the valve (16) when the nucleus prothesis (13) has been filled with fluid and when the filling tube (18) has been screwed off.

3. A nucleus prothesis with an inserting device according to claim 2, characterized in
that the screw is a hexagon socket screw (23).

4. A nucleus prothesis with an inserting device according to one of the claims 1 to 3, characterized in
that the valve (16) merges with the spherical surface of the enveloping body (15) without any steps, when said enveloping body (15) is completely filled with fluid.

5. A nucleus prothesis with an inserting device according to one of the claims 1 to 4, characterized in
that the enveloping body (15) is composed of two layers,
that the outer layer (21) of the enveloping body consists of a high-strength wire cloth or fibre tissue which is compatible with the tissue of the human body, and
that the inner layer (22) of the enveloping body consists of a material which is impermeable to the fluid used.

6. A nucleus prothesis with an inserting device according to claim 5, characterized in
that the outer layer of the enveloping body consists of a titanium tissue (21).

7. A nucleus prothesis with an inserting device according to claim 5 or 6, characterized in
that the inner layer of the enveloping body consists of a fluid-impermeable tissue of plastic material corresponding with regard to its properties to the tissue sold under the trademark of Goretex.

8. A probe for determining the necessary size of a nucleus prothesis which is adapted to be filled with a fluid, in particular according to one of the claims 1 to 9, comprising a cannula (7), an elastic balloon (9) surrounding a front area of said cannula (7) and sealingly secured to said cannula (7), and a fluid supply device (12) used for supplying through the cannula (7) into the interior of the balloon (9) an amount of fluid whose volume can be determined, characterized in
that the outer diameter of the cannula (7) is smaller than the dimensions of an operating access (4) in the anulus fibrosus (3) and
that the ballon (9) can be stretched more easily in the area of the tip of the cannula (7) than in the area where it is secured to said cannula (7).

9. A probe according to claim 10, characterized in
that the fluid supply device consists of a syringe (12) connected to said cannula (7).

10. A probe according to claim 8 or 9, characterized in
that the cannula (7) has a spherical rounded portion (8) on its side facing the balloon (9).

11. A probe according to one of the claim 8 to 10, characterized by
a collar (10) having the shape of a cone or of a truncated cone and surrounding the cannula (7) in the area in which the balloon (9) is fastened thereto.

12. A probe according to claim 11, characterized in
that the collar (10) is provided with at least one passage opening (11) extending in the longitudinal direction of the cannula (7).

## Revendications

1. Prothèse du nucléus avec un dispositif d'introduction (14) pour son introduction dans une chambre intervertébrale,
la prothese du nucléus (13) présentant un élément d'enveloppe (15) pouvant être rempli d'un fluide et qui peut être introduit, dans un état non rempli ou seulement partiellement repoli de fluide, par le dispositif d'introduction, dans la chambre intervertébrale et pouvantt y être rempli de fluide et présentant une soupape (16) disposée dans l'élément d'enveloppe (15),
le dispositif d'introduction (14) présentant un tube d'introduction (17) entourant l'élément d'enveloppe (15), dans son état non rempli ou uniquement partiellement rempli, lors de son introduction dans la chambre intervertébrale (5) et adapté, dans ses dimensions extérieures de section, aux dimensions de l'accès d'opération (4) dans l'anulus fibrosus (3), par lequel l'élément d'enveloppe (15) est positionné dans la chambre intervertébrale (5),
caractérisée en ce qu'à l'intérieur du tube d'introduction (17) est prévu un tube de remplissage (18) qui peut être amené en prise amovible avec l'élément d'enveloppe (15), à l'endroit de sa soupape (16), pour l'expulsion de l'élément d'enveloppe (15) du tube d'introduction (17), dans son état disposé dans la chambre intervertébrale (5) et pour le remplissage de l'élément d'enveloppe (15).

2. Prothèse du nucléus avec un dispositif d'introduction suivant la revendication 1, caractérisé en ce
que le tube de remplissage (18) présente un filet extérieur (19), que la prothèse du nucléus (13) présente, à l'endroit de la soupape (16), un filet intérieur adapté à ce filet extérieur (19), et
que la prothèse du nucléus (13) présente une vis (23) adaptée au filet intérieur (20) avec lequel elle peut être assemblée par vissage pour recouvrir ce dernier et la soupape (16) après remplissage en fluide de la prothèse du nucléus (13) et après dévissage du tube de remplissage (18).

3. Prothèse du nucléus avec un dispositif d'introduction suivant la revendication 2, caractérisée en ce que la vis se présente sous forme d'une vis à six pans creux (23).

4. Prothèse du nucléus avec un dispositif d'introduction suivant l'une des revendications 1 à 3, caractérisée en ce que la soupape (16) se confond, sans paliers, avec la surface sphérique de l'élément d'enveloppe (15), à l'état entièrement rempli de fluide de ce dernier.

5. Prothèse du nucléus avec un dispositif d'introduction suivant l'une des revendications 1 à 4, caractérisée en ce
que l'élément d'enveloppe (15) est réalisé en deux couches,
que la couche extérieure (21) de l'élément d'enveloppe est réalisée en un tissu de fils ou de fibres très solide, compatible avec le tissu du corps humain, et
que la couche intérieure (22) de l'élément d'enveloppe est réalisée en un matériau imperméable au fluide utilisé.

6. Prothèse du nucléus avec un dispositif d'introduction suivant la revendication 5, caractérisée en ce que la couche extérieure de l'élément d'enveloppe est réalisée en un tissu de titane (21).

7. Prothèse du nucléus avec un dispositif d'introduction suivant la revendication 5, caractérisée en ce que la couche intérieure de l'élément d'enveloppe est réalisée en un tissu synthétique imperméable aux fluides qui, en ce qui concerne ses propriétés, correspond au tissu pouvant être obtenu sous le nom commercial de Goretex.

8. Sonde pour la détermination de la grandeur requise d'une prothèse du nucléus pouvant être remplie d'un fluide suivant l'une des revendications 1 à 7, avec une canule (7), un ballon élastique (9), entourant la zone avant de la canule (7), qui est fixé de manière obturante sur la canule (7) et un dispositif d'alimentation de fluide (12) pour l'amenée d'une quantité de fluide pouvant être déterminée en volume, par la canule (7), à l'intérieur du ballon (9), caractérisée en ce
que le diamètre extérieur de la canule (7) est plus petit que la dimension d'un accès d'opération (4) dans l'anulus fibrosus (3), et que le ballon élastique (9) est plus aisément extensible dans la zone de la pointe de la canule que dans la zone de sa fixation sur la canule (7).

9. Sonde suivant la revendication 8, caractérisée en ce que le dispositif d'alimentation de fluide est constitué par une seringue (12) raccordée à la canule (7).

10. Sonde suivant la revendication 8 ou 9, caractérisée en ce que la canule (7) présente, à son extrémité du côté du ballon, un arrondi bombé (8).

11. Sonde suivant l'une des revendications 8 à 10, caractérisée par une manchette conique ou tronconique (10) qui entoure la canule (7) à l'endroit de fixation du ballon (9).

12. Sonde suivant la revendication 11, caractérisée en ce que la manchette (10) présente au moins une ouverture de passage (11) dans le sens de l'extension longitudinale de la canule.
